(19) Europäisches Patentamt
European Patent Office
Office européen des brevets

(11) **EP 3 748 641 A1**

(12) **EUROPEAN PATENT APPLICATION**

(43) Date of publication:
**09.12.2020 Bulletin 2020/50**

(51) Int Cl.:
**G16H 20/30** (2018.01)   **G16H 40/63** (2018.01)
**G16H 50/30** (2018.01)

(21) Application number: **19178424.8**

(22) Date of filing: **05.06.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Aalborg Universitet**
**9220 Aalborg Øst (DK)**

(72) Inventor: **SCHMIDT, Samuel Emil**
**9210 AALBORG SØ (DK)**

(74) Representative: **Brann AB**
**P.O. Box 3690**
**Drottninggatan 27**
**103 59 Stockholm (SE)**

(54) **MULTIPARAMETERIC ESTIMATION OF CARDIORESPIRATORY FITNESS IN SEISMOCARDIOGRAPHY**

(57)    The proposed technology relates to the quantifying of cardiorespiratory fitness. It includes the obtaining (102) of a seismocardiogram (SCG) recorded with an accelerometer (14) configured to measure accelerations and vibrations of the chest wall of a person (18) caused by myocardial movement. Properties of a first signal feature (AC) in the seismocardiogram (SCG) are determined (104), wherein the first signal feature (AC) corresponds to the aortic valve closure (AC) of a heartbeat. A measure indicating cardiorespiratory fitness (VO2max) is then determined (106) based on the properties of first signal feature (AC).

Fig. 2

**Description**

Technical field

The proposed technology relates generally to fitness applications, and particularly to methods and systems for determining an indication of cardiorespiratory fitness.

Background

Cardiorespiratory fitness refers to the ability of the circulatory and respiratory systems to supply oxygen to muscles. The term is generally used for the ability to supply oxygen specifically to skeletal muscles during sustained physical activity, which may therefore be regarded as a subset of cardiovascular fitness.

Cardiorespiratory fitness is affected by physiological parameters, including heart rate, stroke volume, cardiac output, and maximal oxygen consumption. Regular exercise makes these systems more efficient by enlarging the heart muscle, enabling more blood to be pumped with each stroke, and increasing the number of small arteries in trained skeletal muscles.

A common measure of cardiorespiratory fitness is VO2max corresponding to the maximum rate of oxygen consumption measured during an exercise that increases in intensity. Sometimes the measure is normalized by body weight.

There is both a clinical demand and a consumer demand for a low-cost and portable technology that can give an indication of cardiorespiratory fitness, or VO2max.

Seismocardiography is the analysis of SeismoCardioGrams (SCGs) showing sub-audible low-frequency vibrations at the chest wall caused by the beating heart. More generally, seismocardiography typically relates to non-invasive measurement of accelerations in the chest wall produced by myocardial movement. Heart sounds are audible components of the chest wall vibrations that typically are above 40-60 Hz, while SCG vibrations typically are below 25 Hz

A seismocardiogram (SCG) is typically measured using an accelerometer. However, when an accelerometer is used, both low-frequency seismocardiography components and audible components are simultaneously sampled. The signal from the accelerometer is then typically filtered such that it does not contain any audible components.

A SCG reveal different cardiovascular functions by which cardiorespiratory fitness can be determined. For example, seismocardiography is typically suitable for estimation of time intervals between features in the cardiac cycle.

The accelerometer signal is dominated by the high intensity of the low-frequency vibrations caused by the beating heart. If the accelerometer signal is low pass filtered, for example with an upper cutoff of 40 Hz, the influence of heart sounds is removed. In the filtered signal, or SCG signal, dominating features of the heart cycle are the Mitral valve Closure (MC), Isovolumic Movement (IM), Aortic valve Opening (AO), Isovolumic Contraction (IC), the Rapid Ejection (RE), Aortic valve Closure (AC), Mitral valve Opening (MO), and Rapid Filling (RF).

Object

An object of the present invention is to meet the abovementioned need for a technology that can give an indication of cardiorespiratory fitness, and in particular a technology having this capability and that is inexpensive and portable.

Summary

According to a first aspect of the proposed technology, the aforementioned objects are accomplished by a method for quantifying, or determining an indication of, cardiorespiratory fitness. The method comprises: obtaining a seismocardiogram (SCG) recorded with an accelerometer configured to measure accelerations and vibrations of the chest wall of a person caused by myocardial movement. The method further comprises: determining properties of a first signal feature (AC) in, or from, the seismocardiogram (SCG), wherein the first signal feature corresponds to, or relates to, the aortic valve closure (AC) of a heartbeat, or a cardiac cycle. The method further comprises: determining a measure indicating, or determining an indication of, cardiorespiratory fitness (VO2max) based on the properties of the first signal feature.

Here, obtaining a seismocardiogram does not specify the source from which the seismocardiogram is obtained, or how it is obtained. It is understood to encompass both a downloading and an active use of an accelerometer. Alternatively to the specific step of obtaining the seismocardiogram, the first aspect of the proposed technology may be directed to a method for quantifying, or determining an indication of, cardiorespiratory fitness from a seismocardiogram (SCG) recorded with an accelerometer configured to measure accelerations and vibrations of the chest wall of a person caused by myocardial movement.

The proposed technology is centered on the realization that the signal feature in a seismocardiogram (SCG) corresponding to the aortic valve closure (AC) can be used for determining cardiorespiratory fitness. The seismocardiogram may cover a plurality of heartbeats, a single heartbeat, or a portion of a heartbeat, such as a diastolic segment of a heartbeat. It is understood that the first signal feature can correspond to the aortic valve closure (AC) of a single heartbeat, or of an average of heartbeats. A heartbeat is here understood to encompass a complete cardiac cycle. It is further understood that a signal feature may be a complex of joint features, for example a single peak connected to surrounding local minima.

Here, and throughout these specifications, determining, or quantifying, a measure indicating cardiorespiratory fitness is understood to in itself not clearly and unambiguously indicate an abnormal cardiovascular or cardiorespiratory function, condition or structure, or a cardiovascular or cardiorespiratory disorder or disease. Determining a measure indicating cardiorespiratory fitness is however understood to include determining an indication of aerobic fitness, such as maximal oxygen consumption or uptake (VO2max). Naturally, this measure may be influenced indirectly by some abnormal function of the heart, such as a disorder or a disease. However, the measure indicating cardiorespiratory fitness (VO2max) does not point to a specific abnormal function and does not as such constitute a diagnostic measure.

The accelerometer may comprise a piezoelectric element. The signal may represent a voltage generated by the piezoelectric element. Thus, the signal strength or amplitude of a temporal feature may represent a voltage value for the temporal feature. It is understood that the output from the accelerometer does not include any audible components. For example, the recorded signal may have been filtered with a low-pass filter having an upper cut-off that is below 100 Hz, 60 Hz, 40 Hz, 20 Hz, 10 Hz, or 5 Hz.

In a second aspect, a system is provided for quantifying, or determining an indication of, cardiorespiratory fitness. The system comprises: an accelerometer configured to be placed on the chest of a person for measuring accelerations and vibrations of the chest wall of the person caused by myocardial movement. The system further comprises a processor operatively connected to the accelerometer, wherein the processor is configured to perform any of the steps of the method according to the first aspect of the proposed technology.

In a third aspect, a system is provided for quantifying, or determining an indication of, cardiorespiratory fitness, wherein the system comprises: an accelerometer configured to be placed on the chest of a person for measuring accelerations and vibrations of the chest wall of the person caused by myocardial movement and obtaining a seismocardiogram (SCG). The system further comprises: a first determining module determining properties of a first signal feature in, or from, the seismocardiogram (SCG), wherein the first signal feature corresponds to, or relates to, the aortic valve closure (AC) of a heartbeat. The system further comprises a second determining module determining a measure indicating, or determining an indication of, cardiorespiratory fitness (VO2max) based on the properties of the first signal feature.

In a fourth aspect, a computer program product is provided for being used in a system for quantifying, or determining an indication of, cardiorespiratory fitness. The system comprises: an accelerometer configured to be placed on the chest of a person for measuring accelerations and vibrations of the chest wall of the person caused by myocardial movement, and a processor operatively connected to the accelerometer. The computer program product comprises program code instructions configured to, when executed by the processor of the system, cause the processor, or system, to: perform any of the steps of the method according to the first aspect of the proposed technology.

In a fifth aspect, a non-transient memory is provided on which a computer program product according to the fourth aspect of the proposed technology is stored.

Further optional details of the proposed technology are described below.

Detailed description

Determining the properties of the first signal feature may comprise: determining a plurality of segments of the seismocardiogram (SCG), determining one or more noisy segments of the plurality of segments, and discarding the noisy segments. The non-discarded segments comprise the first signal feature and the first signal feature is determined in the non-discarded segments. The segments may comprise diastolic and/or systolic segments. A diastolic segment is understood to be a segment of the seismocardiogram (SCG) that corresponds to the diastolic part of the cardiac cycle. Similarly, a systolic segment is understood to be a segment of the seismocardiogram (SCG) that corresponds to the systolic part of the cardiac cycle.

Determining the properties of the first signal feature may comprise: identifying one or more (first) fiducial points, or reference points, of the first signal feature. The measure indicating cardiorespiratory fitness may further be determined based on the one or more (first) fiducial points. The fiducial points may comprise: the local maxima (ACmax) of the first signal feature, and/or the first local minima (ACmin) immediately before to the local maxima (ACmax). It is understood that the first local minima and the second local minima may correspond to a specific state in the cardiac cycle. For example, the second local minima may correspond to the Mitral valve Opening (MO).

A number of properties, or sub-features, of the first signal feature are described below. The properties are used in determining the measure indicating cardiorespiratory fitness. It has been found that each of the properties contributes to the quantifying, and it is understood that the properties can be used in isolation or in combination with each other. Preferably, all of the sub-features mentioned below are employed in the same method.

Thus, determining properties of the first signal feature may further comprise: determining an amplitude difference (ACPeakToPeak) between the local maxima (ACmax) and the first local minima (ACmin) immediately before to the local maxima (ACmax) of the first signal feature. Determining the measure indicating cardiorespiratory fitness (VO2max) may then further be based on the amplitude difference (ACPeakToPeak).

Additionally or alternatively, determining properties of the first signal feature may further comprise: determining a first time separation (ACTimePeakToPeak) between the local maxima (ACmax) of the first signal feature and the first local minima (ACmin) immediately before to the local maxima (ACmax) of the first signal feature. Determining the measure indicating cardiorespiratory fitness (VO2max) may then further be based on the first time separation (ACTimePeakTo-Peak).

Additionally or alternatively, determining properties of the first signal feature may further comprise: determining a morphology measure (ACMorphology) of the first signal feature. Determining the measure indicating cardiorespiratory fitness (VO2max) may then further be based on the morphology measure (ACMorphology). It is understood that the morphology measure (ACMorphology) is determined in the time domain of the seismocardiogram (SCG). The morphology measure is understood to represent or indicate the shape, contour and/or outline of the first signal feature.

The determining of the morphology measure (ACMorphology) may comprise: determining a first window of the seismocardiogram (SCG), wherein the first window covers, or envelops, the first signal feature. Determining the morphology measure (ACMorphology) may then be based on the time domain of the first window.

It is understood that the first window may be determined from an average diastolic segment. Alternatively, the first window may be determined for each diastolic segments of the seismocardiogram (SCG). An average first window may then be determined before determining the morphology measure (ACMorphology).

In a preferred embodiment, individual morphology measures are determined for each first window, or for each diastolic segment, and the morphology measure (ACMorphology) is determined as an average of the individual morphology measures.

The width of the first window may be predetermined. Based on the time domain of the first window is understood to encompass the morphology measure (ACMorphology) being based on changes in signal value or amplitude over time in the first window. The first window may have a width in the range 200 ms to 500 ms, 250 ms to 450 ms, or 300 ms to 400 ms. This means that the technology can be applied at heart rates up to about 100 beats per minute.

Determining the first window may comprise: identifying a fiducial point forming part of the first signal feature, and positioning the first window relative to the fiducial point. The fiducial point may be the local maxima (ACmax) of the first signal feature. It is understood that the local maxima (ACmax) of the first signal feature may be the local maxima (ACmax) of a diastolic segment containing the first signal feature. The first window may start in the range 40 ms to 60 ms, or at 50 ms, before the fiducial point and end in the range 200 ms to 500 ms, 300 ms to 400 ms, or at 350 ms after the fiducial point.

Determining the morphology measure (ACMorphology) may comprise: providing, or loading, a first machine learning model trained on seismocardiogram (SCG) windows operationally similar to the first window for determining a morphology measure, and determining the morphology measure (ACMorphology) by inputting the first window in the first machine learning model. The term "operationally similar" is understood to encompass each of the seismocardiogram (SCG) windows that the model is trained on being defined or generated in the same manner, or comprising similar features, as the first window.

Determining properties of the first signal feature may further comprise: determining a frequency measure (ACFrequency) of the first signal feature, and determining the measure indicating cardiorespiratory fitness (VO2max) may further be based on the frequency measure (ACFrequency). The frequency measure (ACFrequency) may be determined in the frequency domain of the seismocardiogram (SCG).

Determining the frequency measure (ACFrequency) may comprise: determining a second window of the seismocardiogram (SCG), wherein the second window covers, or envelops, the first signal feature. Determining the frequency measure (ACFrequency) may then be based on the frequency domain of the second window.

Determining the frequency measure (ACFrequency) may further comprise: determining the power spectrum density, or spectral density, of the second window. Determining the frequency measure (ACFrequency) may then further be based on the power spectrum density.

Similar to the first window, it is understood that the second window, or power spectrum density, may be determined for an average diastolic segment. Alternatively, the second window, or power spectrum density, may be determined for each diastolic segments, or second window, of the seismocardiogram (SCG). In a preferred embodiment, individual power spectrum densities are determined for each first window, or for each diastolic segment and an individual frequency measure is determined from each individual power spectrum density, and the frequency measure (ACFrequency) is determined as an average of the individual frequency measures.

The second window may have any of the features of the first window described above. The first window and the second window may be the same, or have the same features or properties. Above, based on the frequency domain of the second window is understood to encompass the frequency measure (ACFrequency) being based on changes in signal value or amplitude over frequency in the first window.

Determining the frequency measure (ACFrequency) may comprise: providing, or loading, a second machine learning model trained on the frequency domain, or power spectrum density, of seismocardiogram (SCG) windows operationally similar to the second window for determining the frequency measure (ACFrequency). It may further comprise: determining the frequency measure (ACFrequency) by inputting the second window in the second machine learning model. By the term "operationally similar" is understood that the frequency domain, or power spectrum density, of each seismocardiogram (SCG) window is defined or generated in the same manner, or has the same general properties, as the frequency domain, or power spectrum density, of the second window.

The method according to the first aspect of the proposed technology may further comprise: determining a heart rate variability (HRV). Determining the measure indicating cardiorespiratory fitness (VO2max) may then further be based on the heart rate variability (HRV). The heart rate variability (HRV) may be determined from the seismocardiogram (SCG), or from an electrocardiogram (ECG) obtained simultaneously to, or in connection with, the seismocardiogram (SCG).

Determining the heart rate variability (HRV) may further comprise: determining a plurality of time intervals between fiducial points of the same type in succeeding heart beats, and determining the heart rate variability (HRV) as, or based on, the standard deviation of the plurality of time intervals.

The method according to the first aspect of the proposed technology may further comprise: determining properties of a second signal feature in the seismocardiogram (SCG), wherein the second signal feature is different, or disjoint, from the first signal feature. Determining the measure indicating cardiorespiratory fitness (VO2max) may then further be based on the properties of the second signal feature. It is understood that the second signal feature can be from a single heartbeat or constitute an average of several heartbeats, as described above for the first signal feature.

Determining the properties of the second signal feature may comprise: determining a plurality of systolic segments of the seismocardiogram (SCG), and discarding noisy systolic segments, wherein the non-discarded systolic segments comprises the second signal feature. A systolic segment is understood to be a segment of the seismocardiogram (SCG) that corresponds to the systolic part of the cardiac cycle.

The second signal feature may correspond to the mitral valve closure (MC) and/or the aortic valve opening (AO) of a heart cycle. Determining the properties of the second signal feature may comprise: identifying one or more (second) fiducial points, or reference points, of the second signal feature. The measure indicating cardiorespiratory fitness may further be determined based on the one or more (second) fiducial points. The fiducial points may comprise: the zero crossing of the mitral valve closure (MC) and the first local maxima (AOmax) of the aortic valve opening (AO) after the mitral valve closure (MC).

Determining properties of the second signal feature comprises may then comprise: determining a second time separation (SysTimeMCToAO) between the zero crossing of the mitral valve closure (MC) and the first local maxima (AOmax) of the aortic valve opening (AO) after the mitral valve closure (MC). Determining the measure indicating cardiorespiratory fitness (VO2max) may then further be based on the second time separation (SysTimeMCToAO).

In the proposed method, determining a measure indicating cardiorespiratory fitness (VO2max) may further be based on a trained machine learning model. Preferably, the method is based on at least three machine learning models, a first for determining the morphology measure (ACMorphology), a second for determining the frequency measure (ACFrequency), and a third for determining the measure indicating cardiorespiratory fitness (VO2max) as such. This means that the latter model may include the results of the two former models.

Brief description of the drawings

A more complete understanding of the abovementioned and other features and advantages of the present invention will be apparent from the following detailed description of the drawings, wherein:

Fig. 1 is a schematic illustration of an embodiment of a system for determining an indication of cardiorespiratory fitness,
Fig. 2 is a flow chart illustrating the basic steps of a method employed in the system described in relation to Fig.1,
Figs. 3a-b are flow charts illustrating different embodiments for determining a morphology measure,
Figs. 4a-b are flow charts illustrating different embodiments for determining a frequency measure,
Fig. 5 is a graph showing an electrocardiogram of a heart cycle,
Fig. 6 is graph showing a seismocardiogram of a heart cycle recorded simultaneously to the electrocardiogram of Fig. 5, and
Fig. 7 is graph showing a window overlaid on the seismocardiogram of Fig. 6.

Description of the drawings

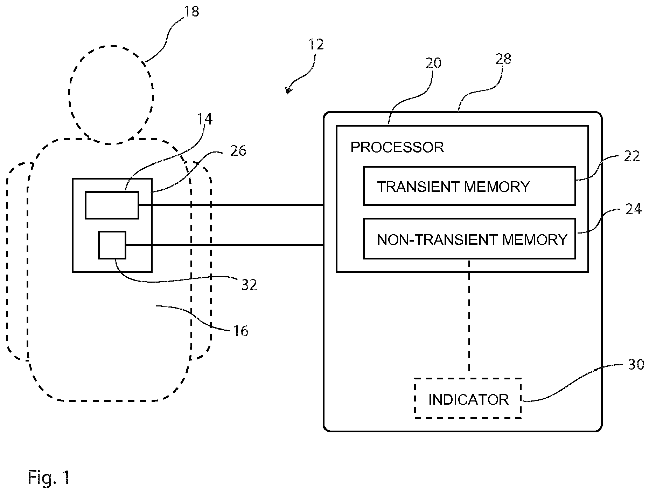

Fig.1 schematically illustrates an embodiment of a system 12 for quantifying cardiorespiratory fitness. The system 12 has an accelerometer 14 in the form of a piezoelectric element that can be placed on the chest of a person 18 and for measuring vibrations of the chest wall caused by movements of the heart. A processor 20 is connected to the accelerometer 14. The processor 20 has a transient memory 22 which can store a signal received from the accelerometer 14, and by which it can execute program code instructions. The system 12 comprises a support 26 that supports the accelerometer 14 and a housing 28 that accommodates the processor 20. The system 12 also has a non-transient memory 24 storing program code instructions for the processor 20. For example, the system 12 as a whole can be an integral part of a smart-phone, or all parts except the accelerometer 20 and the support 26 can form part of a smart-phone. In one embodiment, the accelerometer 14 is an integrated accelerometer of a smart-phone.

In one embodiment of the system 12, it additionally has an indicator 30 operatively connected with the processor 20. The indicator 30 can, for example, have an LCD display, or the like, that can display output information from the processor 20, such as a number indicating a measure of cardiorespiratory fitness.

The system also comprises electrocardiogram electrodes 32 (two leads plus ground) supported by the support 26. The electrodes 32 are connected to the processor 20.

The primary function of the accelerometer 14 is to sample a seismocardiogram (SCG) for further analysis. The primary function of the electrodes 32 is to sample an electrocardiogram (ECG) that is used for segmentation of the seismocardiogram (SCG).

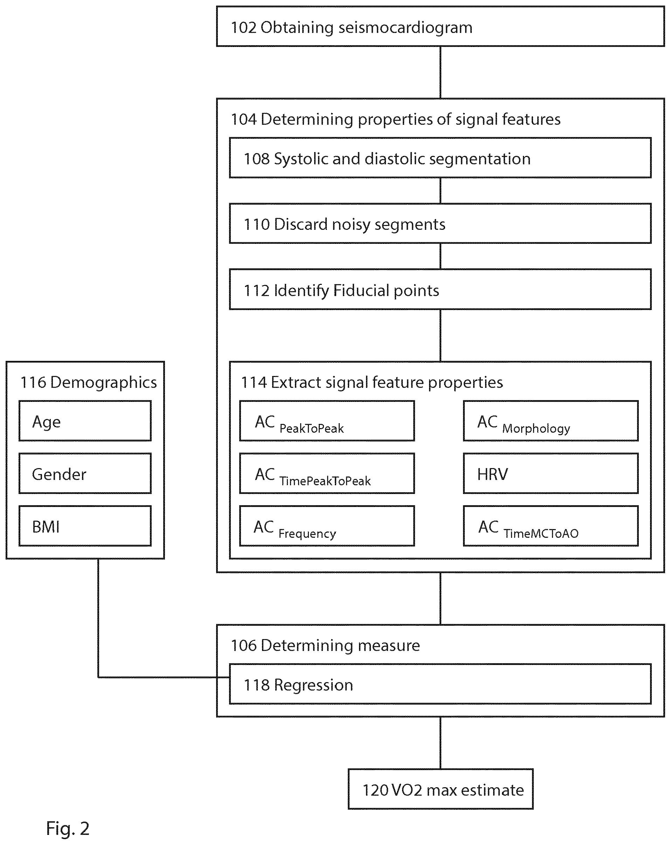

The program code instructions in the non-transient memory 24 cause the processor 20 to perform a method that is shown in Fig.2. A seismocardiogram (SCG) is obtained 102 with the accelerometer 14 placed on the chest of a person 18. In an alternative embodiment, the SCG is downloaded from a server to which it previously has been uploaded. Properties of two signal features are determined 104 in the seismocardiogram (SCG). How this is achieved is described in detail below. A measure indicating cardiorespiratory fitness (VO2max) is then determined 106 based on the properties of first signal feature.

A plurality of systolic and diastolic segments is determined 108 in the SCG. This is achieved by an automated segmentation method using an electrocardiogram (ECG) simultaneously acquired by the electrodes 32 as reference. In alternative embodiments, the segmentation is based on the SCG as such, e.g. similar to the technology described in US8235912 (B2). Noisy segments are then discarded 110, for example as described in WO2017216375(A1).

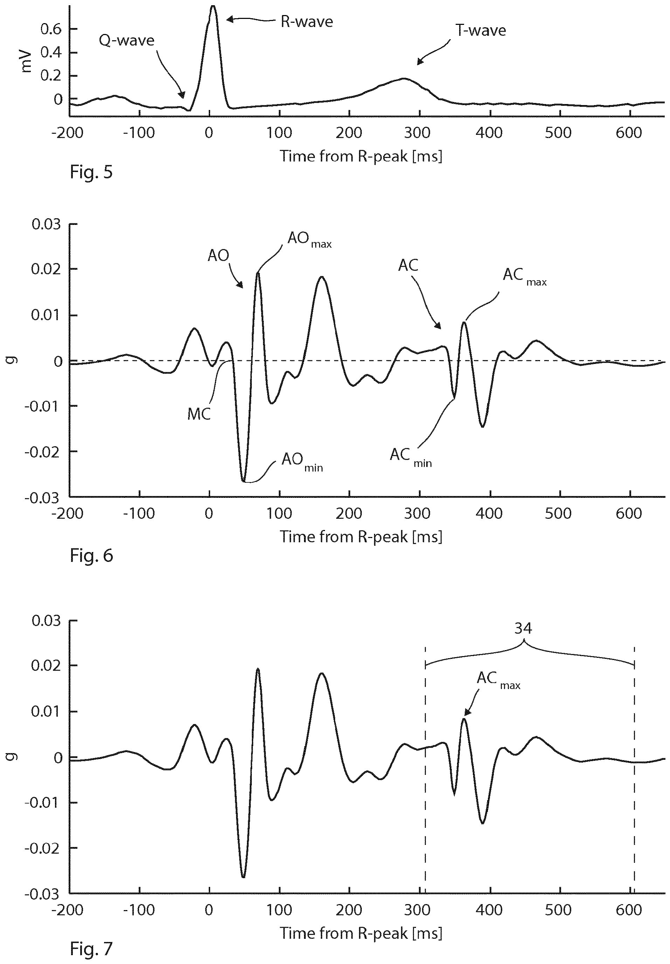

Fig. 5 shows an electrocardiogram (ECG) with the signal from the electrodes 32 in the unit Volt (mV) as function of time (ms). The time has been reset with respect to the peak of the R-wave. Fig. 6 shows a simultaneously recorded seismocardiogram (SCG) with the accelerometer signal in the unit of gravitational force equivalents (g) as a function of time (ms) reset in the same manner as the electrocardiogram (ECG) .

Each of the resulting diastolic segments includes or comprises a first signal feature corresponding to the aortic valve closure (AC) of a single heartbeat. Similarly, each of the resulting systolic segments includes a second signal feature corresponding to the combined mitral valve closure (MC) and the aortic valve opening (AO) of a heart cycle.

Fiducial points are then identified 112 for respective signal feature in the systolic and diastolic segments, similar to identifications described in WO2017216375(A1). The following fiducial points are determined in each diastolic segment, or for each first signal feature:

- the local maxima ($AC_{max}$) of the aortic valve closure (AC), and
- the first local minima ($AC_{min}$) immediately before to the local maxima ($AC_{max}$).

The following fiducial points are determined in each systolic segment, or for each second signal feature:

- the zero crossing of the mitral valve closure (MC), and
- the first local maxima (AOmax) of the aortic valve opening (AO) after the mitral valve closure (MC).

The abovementioned fiducial points are indicated in Fig. 6. Properties of the signal features are then determined 114 based at least in part on the fiducial points.

A first property of the first signal feature corresponding to the aortic valve closure (AC) is the amplitude difference (ACPeakToPeak) between the local maxima (ACmax) and the first local minima (ACmin). A measure (ACPeakToPeak) representing the property is determined from the mean of the diastolic segments. A second property of the same signal feature is the (first) time separation (ACTimePeakToPeak) between the local maxima (ACmax) and the first local minima (ACmin) immediately before to the local maxima (ACmax). A measure (ACTimePeakToPeak) representing the property is determined from the mean of the diastolic segments normalized to a standard deviation of one. A third property is the morphology of the of the first signal feature represented by a morphology measure ($AC_{Morphology}$). A fourth property is a frequency measure ($AC_{Frequency}$) of the of the first signal feature. The determining of the latter two properties is further described below.

When determining the morphology measure ($AC_{Morphology}$), a (first) window 34 is identified in the seismocardiogram (SCG). The window 34 starts 50 ms before the local maxima (ACmax) and ends 250 ms after the local maxima (ACmax). A corresponding window 34 is indicated in Fig. 7 showing the seismocardiogram (SCG) of Fig. 6.

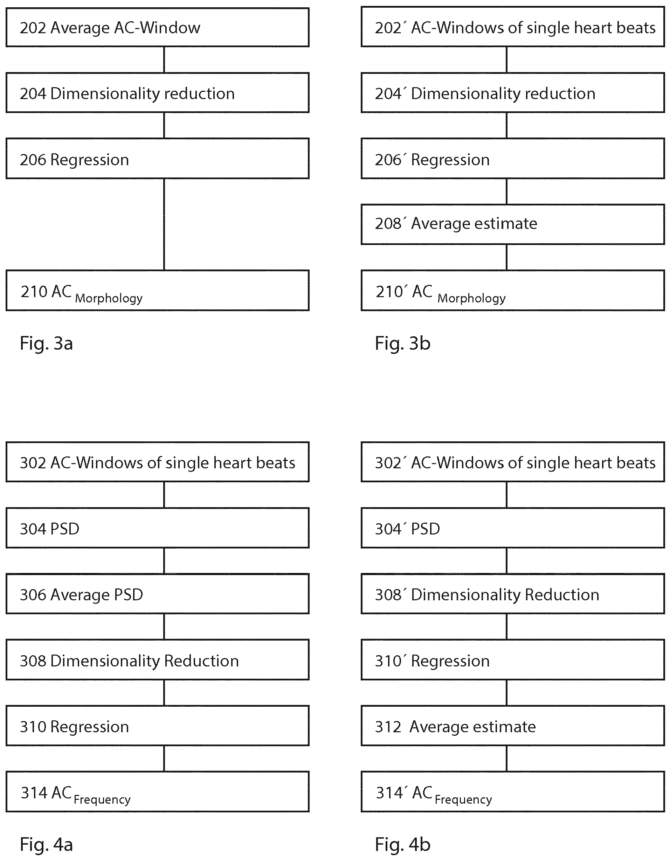

The morphology measure ($AC_{Morphology}$) is determined using an early averaging. The steps relating to the early averaging are illustrated in Fig. 3a. An average diastolic segment is determined and the window 34 is then identified 202 in the average diastolic segment. The amplitudes in the window 34 are normalized to a standard deviation of one to reduce the influence of amplitude variations. A dimensionality reduction 204 is then performed using principal component analysis (PCA) conducted on the window 34 of the average diastolic segment for identifying principal components. In an alternative embodiment, the dimensionality reduction 204 is performed using an auto encoder (AE) with the window 34 of the diastolic segment as input and constructed such that the auto encoder (AE) compresses the window into a few nodes in a neural network, for example 10 nodes. A regression 206 is then performed using a linear regression function with the principal components, or nodes, as input. In alternative embodiments, the regression function is based on neural network regression, convolutional neural network regression, or Support vector machine regression. The regression 206 results in a morphology measure ($AC_{Morphology}$) 210 representing or quantifying the shape of the first signal feature, or the complex of the seismocardiogram (SCG) relating to the aortic valve closure (AC).

In an alternative embodiment, the morphology measure ($AC_{Morphology}$) is determined using a late averaging. The steps relating to the late averaging are illustrated in Fig. 3b. The window 34 is identified 202' in each diastolic segment. The amplitudes in each window 34 are normalized to a standard deviation of one to reduce the influence of amplitude variations. A dimensionality reduction 204' is then performed using principal component analysis (PCA) conducted on a matrix of the windows 34 for identifying principal components. In an alternative embodiment, the dimensionality reduction 204' is performed using an auto encoder (AE) with a matrix of the windows 34 as input compressing them into a number of nodes, such as ten, in a neural network. A regression 206 is then performed using a linear regression function with the principal components, or nodes, as input. In alternative embodiments, the regression function is based on neural network regression, convolutional neural network regression, or support vector machine regression. The regression 206' results in an individual measure for each window of a diastolic segment. An average over the individual measures is calculated 208' yielding the morphology measure ($AC_{Morphology}$) 210'.

When determining the frequency measure ($AC_{Frequency}$), the same windows 34 in the diastolic segments are used as when determining the morphology measure ($AC_{Morphology}$).

The frequency measure ($AC_{Frequency}$) is determined using an early averaging. The steps relating to the early averaging are illustrated in Fig. 4a. The window 34 is identified 302' in each average diastolic segment. The amplitudes in the windows 34 are normalized to a standard deviation of one to reduce the influence of amplitude variations. The power spectrum density (PSD) is then determined 304 in each window 34. An average power spectrum density (PSD) is determined 306, which is used in a dimensionality reduction 308 similar to the dimensionality reductions 204 described above in relation to the early averaging approach for determining the morphology measure ($AC_{Morphology}$). A regression 310 is then performed using the resulting principal components, or nodes, as input, similar to the regressions 206 described in relation to the abovementioned early averaging approach. The regression 310 results in a frequency measure ($AC_{Frequency}$) 314 representing or quantifying the properties in the frequency domain of the average window, and in extension of the first signal feature.

In an alternative embodiment, the frequency measure ($AC_{Frequency}$) is determined using a late averaging. The steps relating to the late averaging are illustrated in Fig. 4b. The window 34 is identified 302' in each diastolic segment. The amplitudes in each window 34 are normalized to a standard deviation of one to reduce the influence of amplitude variations. The power spectrum density (PSD) is determined 304 in each window 34. Instead of calculating an average power spectrum density (PSD), a dimensionality reduction 308' similar to the dimensionality reductions 204' described above in relation to the late averaging approach for determining the morphology measure ($AC_{Morphology}$) is performed on a matrix of the determined power spectrum densities. A regression 310' is then performed using the principal components, or nodes, for each window as input, similar to the regressions 206' described in relation to the abovementioned late averaging approach. The regression 310' results in a number of individual measures, each corresponding to a window of a diastolic segment. A frequency measure ($AC_{Frequency}$) 314' is then determined as the mean 312, or in an alternative embodiment the median, of the individual measures.

The abovementioned four properties of the first signal feature are used in the input for determining 106 the measure indicating cardiorespiratory fitness (VO2max). Additionally, the heart rate variability (HRV) and a property of the second signal feature corresponding to the mitral valve closure (MC) and the aortic valve opening (AO) of a heart cycle are used as input.

The heart rate variability (HRV) is determined by first identifying succeeding heart beats in the seismocardiogram (SCG), and then calculating as the length of a the time interval between the same fiducial points, such as the local maxima ($AC_{max}$) of the aortic valve closure (AC), in succeeding heart beats.

The property of the second signal feature is the time separation (SysTimeMCToAO) between the zero crossing of the mitral valve closure (MC) and the first local maxima (AOmax) of the aortic valve opening (AO) after the mitral valve closure (MC). A measure representing the property is determined from the mean of the diastolic segments normalized to a standard deviation of one.

Demographic measures are also provided 116 representing the age, gender, and body-mass index of the person.

All the above mentioned measures are used as input in a multi-parametric regression 118 for determining 106 the measure indicating cardiorespiratory fitness (VO2max) 120. Effectively, in the preferred embodiment the regression is represented by:

$$VO2maxPrKG = \omega1 \; ACPeakToPeak + \omega2 \; ACTimePeakToPeak + \omega3 \; ACFrequency + \omega4 \; ACMorphology + \omega5 \; HRV + \omega6 \; SysTimeMCToAO + \omega7 \; Age + \omega8 \; Gender + \omega9 \; BMI$$

In alternative embodiments, fewer measures are used in the regression 118. Here, the measure indicating cardiorespiratory fitness (VO2maxPrKG) is normalized with respect to bodyweight.

Proof-of-concept

The proposed technology has been validated in 145 measurements from 133 subjects. In each measurement, a seismocardiogram was recorded and immediately afterwards the subject underwent traditional VO2max test. The latter was considered the golden standard and a performance measure was determined as the correlation between the VO2max predicted by the proposed technology and the golden standard VO2max. In the final validation the standard error of estimate (SEE) was used to evaluate the error between the predicted and golden standard VO2max.

The Validation of the individual features was conducted using a 5-fold cross validation, where 3-folds were used for training, one fold for validation and one fold for test. The validation of the final score was conducted using 5 times repetition of 10 fold-cross validation.

For the frequency measure (ACFrequency), the best performing feature extraction method was the linear regression after PCA using early averaging, see table 1. For the morphology measure (ACMorphology), the best performing feature extraction method was the linear regression after PCA using late averaging, see table 2.

The correlations between the reference, or golden standard, VO2max and the predicted VO2max, or determined measure indicating cardiorespiratory fitness (VO2max), are shown in table 3. Results including and excluding the frequency measure (ACFrequency) and the morphology measure (ACMorphology) are shown. It can be concluded from table 3 that both these measures increase the correlation of the reference VO2max and the predicted VO2max. It can be concluded that the addition of both the frequency measure (ACFrequency) and the morphology feature (ACMorphology) improved performance of the proposed method for determining a measure (VO2max) indicating cardiorespiratory fitness, or VO2max.

Table 1: Correlation between the frequency measure ($AC_{Frequency}$) and the measure indicating cardiorespiratory fitness (VO2max) depending on the use of Dimensionality Reduction and regression method

| Dimensionality Reduction | None | | AE | | PCA | |
|---|---|---|---|---|---|---|
| Regression method | Linear Regression | Neural Network | Linear Regression | Neural Network | Linear Regression | Neural Network |
| Early avg. | **0.737** | 0.577 | 0.698 | 0.683 | **0.740** | 0.681 |
| Late avg. | 0.736 | 0.682 | 0.699 | 0.734 | 0.709 | 0.656 |

Table 2: Correlation between the morphology measure ($AC_{Morphology}$) and the measure indicating cardiorespiratory fitness (VO2max) depending on the use of Dimensionality Reduction and regression method

| Dimensionality Reduction | None | | AE | | PCA | |
|---|---|---|---|---|---|---|
| Regression method | Linear Regression | Neural Network | Linear Regression | Regressi on method | Linear Regression | Neural Network |

(continued)

| Dimensionality Reduction | None | | AE | | PCA | |
|---|---|---|---|---|---|---|
| Early avg. | 0.651 | 0.446 | 0.679 | 0.714 | 0.717 | 0.508 |
| Late avg. | 0.715 | 0.663 | 0.657 | 0.738 | **0.744** | 0.659 |

Table 3: Correlation between the frequency measure ($AC_{Frequency}$), the the morphology measure ($AC_{Morphology}$), and reference VO2max depending on the use of dimensionality reduction and regression method.

| Properties/ measures | Age Gender Weight $AC_{TimePeakToPeak}$ $AC_{PeakToPeak}$ $Sys_{TimeMCToAO}$ | Age Gender Weight $AC_{TimePeakToPeak}$ $AC_{PeakToPeak}$ $Sys_{TimeMCToAO}$ | Age Gender Weight $AC_{TimePeakToPeak}$ $AC_{PeakToPeak}$ $Sys_{TimeMCToAO}$ | Age Gender Weight $AC_{TimePeakToPeak}$ $AC_{PeakToPeak}$ $Sys_{TimeMCToAO}$ |
|---|---|---|---|---|
| | | $AC_{Frequency}$ | $AC_{Morphology}$ | $AC_{Morphology}$ $AC_{Frequency}$ |
| *10-fold cross validation* | | | | |
| Correlation to VO2max | 0.8130 | 0.8227 | 0.8340 | 0.8365 |
| SEE ((mL/min) /kg) | 5.9283 | 5.7879 | 5.6128 | 5.5755 |
| *Full dataset* | | | | |
| Correlation to VO2max | 0.8276 | 0.8617 | 0.8594 | 0.8678 |
| SEE ((mL/min) /kg) | 5.7059 | 5.1865 | 5.2002 | 5.0510 |

**Claims**

1. A method (100) for quantifying cardiorespiratory fitness comprising:

- obtaining (102) a seismocardiogram (SCG) recorded with an accelerometer (14) configured to measure accelerations and vibrations of the chest wall of a person (18) caused by myocardial movement,
- determining (104) properties of a first signal feature (AC) in the seismocardiogram (SCG), wherein the first signal feature (AC) corresponds to the aortic valve closure (AC) of a heartbeat,
- determining (106) a measure indicating cardiorespiratory fitness (VO2max) based on the properties of first signal feature (AC).

2. The method according to claim 1, wherein determining (104) the properties of the first signal feature (AC) comprises:

- determining (108) a plurality of diastolic segments of the seismocardiogram (SCG), and
- discarding (110) noisy diastolic segments, wherein the non-discarded diastolic segments comprise the first signal feature (AC).

3. The method according to claim 2, wherein determining (104) the properties of the first signal feature (AC) comprises:

- identifying (112) one or more fiducial points of the first signal feature (AC),

and the measure indicating cardiorespiratory fitness is further determined based on the one or more fiducial points, wherein
the fiducial points comprise: the local maxima (ACmax) of the first signal feature (AC), and/or the first local minima (ACmin) immediately before to the local maxima (ACmax).

4. The method according to any of the claims 1 to 3, wherein determining (104) properties of the first signal feature (AC) comprises:

- determining (114) an amplitude difference (ACPeakToPeak) between the local maxima (ACmax) and the first local minima (ACmin) immediately before the local maxima (ACmax) of the first signal feature (AC), and
- determining (106) the measure indicating cardiorespiratory fitness (VO2max) is further based on the amplitude difference (ACPeakToPeak).

5. The method according to any of the claims 1 to 4, wherein determining (104) properties of the first signal feature (AC) comprises:

- determining (114) a first time separation (ACTimePeakToPeak) between the local maxima (ACmax) and the first local minima (ACmin) immediately before the local maxima (ACmax) of the first signal feature (AC), and
- determining (106) the measure indicating cardiorespiratory fitness (VO2max) based on the first time separation (ACTimePeakToPeak).

6. The method according to any of the claims 1 to 5, wherein determining (104) properties of the first signal feature (AC) comprises:

- determining (114) a morphology measure (ACMorphology) of the first signal feature (AC), and
- determining (106) the measure indicating cardiorespiratory fitness (VO2max) is further based on the morphology measure ($AC_{Morphology}$),

wherein
determining (114) the morphology measure (ACMorphology) comprises:

- determining (202, 202') a first window (34) of the seismocardiogram (SCG), wherein the first window (34) covers, or envelops, the first signal feature (AC), and
- determining (210, 210') the morphology measure (ACMorphology) based on the time domain of the first window (34).

7. The method according to claim 6, wherein the first window (34) has a width in the range 200 ms to 500 ms, 250 ms to 450 ms, or 300 ms to 400 ms.

8. The method according to any of the claims 1 to 7, wherein determining (104) properties of the first signal feature (AC) comprises:

- determining (114) a frequency measure (ACFrequency) of the first signal feature (AC), and
- determining (106) the measure indicating cardiorespiratory fitness (VO2max) is further based on the frequency measure (ACFrequency),

wherein
determining (104) the frequency measure (ACFrequency) comprises:

- determining (302, 302') a second window (34) of the seismocardiogram (SCG), wherein the second window (34) covers, or envelops, the first signal feature (AC), and
- determining (314, 314') the frequency measure (ACFrequency) based on the frequency domain of the second window (34).

9. The method according to any of the claims 1 to 8 further comprising:

   - determining (114) a heart rate variability (HRV), and
   - determining (106) the measure indicating cardiorespiratory fitness (VO2max) is further based on the heart rate variability (HRV).

10. The method according to any of the claims 1 to 9 further comprising:

    - determining (104) properties of a second signal feature (MC+AC) in the seismocardiogram (SCG), wherein the second signal feature (MC+AC) is different, or disjoint, from the first signal feature (AC), and
    - determining (106) the measure indicating cardiorespiratory fitness (VO2max) is further based on the properties of the second signal feature (MC+AC),

    wherein
    the second signal feature (MC+AC) corresponds to the mitral valve closure (MC) and/or the aortic valve opening (AO) of a heart cycle.

11. The method according to claim 10, wherein determining (104) properties of the second signal feature (MC+AC) further comprises:

    - determining (114) a second time separation (SysTimeMCToAO) between the zero crossing of the mitral valve closure (MC) and the first local maxima (AOmax) of the aortic valve opening (AO) after the mitral valve closure (MC), and
    - determining (106) the measure indicating cardiorespiratory fitness (VO2max) is further based on the second time separation (SysTimeMCToAO).

12. The method according to any of the claims 1 to 11, wherein determining (106) a measure indicating cardiorespiratory fitness (VO2max) is further based on a trained machine learning model.

13. A system (12) for quantifying, or determining an indication of, cardiorespiratory fitness comprising:

    (A) an accelerometer (14) configured to be placed on the chest of a person (18) for measuring accelerations and vibrations of the chest wall of the person (18) caused by myocardial movement, and
    (B) a processor (20) operatively connected to the accelerometer (14), wherein the processor (20) is configured to perform any of the steps of the method according to claims 1 to 12.

14. A computer program product for being used in a system (12) for quantifying, or determining an indication of, cardiorespiratory fitness, wherein the system comprises: (A) an accelerometer (14) configured to be placed on the chest of a person (18) for measuring accelerations and vibrations of the chest wall of the person (18) caused by myocardial movement, and (B) a processor (20) operatively connected to the accelerometer, wherein the computer program product comprising program code instructions configured to, when executed by the processor (20) of the system (12): perform any of the steps of the method according to claims 1 to 12.

15. A non-transient memory (24) on which a computer program product according to claim 14 is stored.

Fig. 1

102 Obtaining seismocardiogram

104 Determining properties of signal features

108 Systolic and diastolic segmentation

110 Discard noisy segments

112 Identify Fiducial points

116 Demographics

Age

Gender

BMI

114 Extract signal feature properties

AC $_{PeakToPeak}$

AC $_{Morphology}$

AC $_{TimePeakToPeak}$

HRV

AC $_{Frequency}$

AC $_{TimeMCToAO}$

106 Determining measure

118 Regression

120 VO2 max estimate

Fig. 2

| 202 Average AC-Window |
| 204 Dimensionality reduction |
| 206 Regression |
| 210 AC Morphology |

Fig. 3a

| 202´ AC-Windows of single heart beats |
| 204´ Dimensionality reduction |
| 206´ Regression |
| 208´ Average estimate |
| 210´ AC Morphology |

Fig. 3b

| 302 AC-Windows of single heart beats |
| 304 PSD |
| 306 Average PSD |
| 308 Dimensionality Reduction |
| 310 Regression |
| 314 AC Frequency |

Fig. 4a

| 302´ AC-Windows of single heart beats |
| 304´ PSD |
| 308´ Dimensionality Reduction |
| 310´ Regression |
| 312 Average estimate |
| 314´ AC Frequency |

Fig. 4b

Fig. 5

Fig. 6

Fig. 7

Europäisches
Patentamt

European
Patent Office

Office européen
des brevets

# EUROPEAN SEARCH REPORT

Application Number

EP 19 17 8424

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/216375 A1 (ACARIX AS [DK]) 21 December 2017 (2017-12-21) * page 2, line 25 - page 21, line 10 * ----- | 1-15 | INV. G16H20/30 G16H40/63 G16H50/30 |
| X | US 2014/221859 A1 (ALBERT DAVID E [US]) 7 August 2014 (2014-08-07) * paragraph [0016] - paragraph [0040] * * paragraph [0049] - paragraph [0122] * ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 11 December 2019 | Menschner, Philipp |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 17 8424

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2019

| Patent document cited in search report | Publication date | Patent family member(s) | Publication date |
|---|---|---|---|
| WO 2017216375 A1 | 21-12-2017 | AU 2017285334 A1 | 06-12-2018 |
| | | CA 3026972 A1 | 21-12-2017 |
| | | CN 109310370 A | 05-02-2019 |
| | | CN 109310371 A | 05-02-2019 |
| | | EP 3257441 A1 | 20-12-2017 |
| | | EP 3471609 A1 | 24-04-2019 |
| | | EP 3471610 A1 | 24-04-2019 |
| | | JP 2019523111 A | 22-08-2019 |
| | | US 2019175072 A1 | 13-06-2019 |
| | | US 2019336013 A1 | 07-11-2019 |
| | | WO 2017216374 A1 | 21-12-2017 |
| | | WO 2017216375 A1 | 21-12-2017 |
| US 2014221859 A1 | 07-08-2014 | US 2014066798 A1 | 06-03-2014 |
| | | US 2014221859 A1 | 07-08-2014 |
| | | WO 2014036436 A1 | 06-03-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8235912 B2

- WO 2017216375 A1